# FASCICULE DE BREVET EUROPEEN

(11) **EP 0 538 153 B1**
(45) Date de publication et mention de la délivrance du brevet: **02.11.1995**
(21) Numéro de dépôt: 92420338.3
(22) Date de dépôt: 29.09.1992
(51) Int. Cl.: A61B 17/15

(54) **Ancillaire modulaire pour la mise en place d'une prothèse de genou**
Modulare Hilfseinrichtung zum Einsetzen einer Knieprothese
Modular auxiliary instrument for the implantation of a knee prosthesis

(30) Priorité: 01.10.1991 FR 9112283
(43) Date de publication de la demande: 21.04.1993
(73) Titulaire: IMPACT, F-01800 Charnoz (FR); Collomb, Jean, F-26800 Porte Les Valence (FR); Majou, Claude, F-01800 Meximieux (FR)
(72) Inventeur: Collomb, Jean, F-26800 Porte les Valence (FR); Majou, Claude, F-01800 Meximieux (FR)
(74) Mandataire: Laurent, Michel

(56) Documents cités:
- EP-A- 0 243 109
- EP-A- 0 380 451
- FR-A- 2 664 157

## Description

L'invention concerne un ancillaire modulaire, destiné à permettre la mise en place d'une prothèse de genou.

La mise en place d'une prothèse de genou suppose une préparation chirurgicale préalable, consistant à préparer l'extrémité inférieure du fémur, destiné à recevoir la prothèse. De manière connue, ce travail préparatoire, consistant principalement à réaliser les surfaces de ladite extrémité avant de les rendre complémentaires avec les surfaces internes des prothèses de genou, est effectué au moyen d'un ancillaire. Les ancillaires connus à ce jour sont typiquement constitués d'au moins deux cents pièces, et s'avèrent de fait difficiles à manipuler et sont en outre particulièrement coûteux.

Le préambule de la revendication indépendante mentionne les caractéristiques connues du document EP-A-0380451 qui décrit un appareil pour la résection du fémur et pour la mise en place d'une prothèse d'articulation du genou.

L'objet de l'invention est de proposer un ancillaire modulaire, universel, compact, permettant de déterminer de manière précise l'importance en millimètre des coupes à effectuer au niveau de l'extrémité inférieure du fémur.

Cet ancillaire modulaire pour la mise en place d'une prothèse de genou se compose :
- d'une glissière rectiligne, percée en son centre d'une lumière également rectiligne, parallèle à la dimension principale de la glissière, recevant un coulisseau fixable réversiblement droit/gauche, destiné à servir de guide pour une tige intramédullaire insérée dans le canal médullaire du fémur, et permettant d'assurer la mise en place de l'ancillaire ;
- d'un gabarit, fixé à l'extrémité inférieure de la glissière, et comportant deux éléments plans perpendiculaires à la glissière, destinés à venir prendre appui sur les condyles postérieurs du fémur lorsque l'articulation est à 90 degrés de flexion ;
- d'un guide de coupe antérieure, coulissable sur la glissière au dessus du coulisseau, ledit guide comportant :
   . au moins une fente traversante perpendiculaire à la glissière ;
   . un palpeur cortical, ménagé à l'extrémité libre d'une tige de guidage, dont l'autre extrémité est solidaire du centre du guide de coupe antérieure, ladite tige de guidage s'étendant parallèlement au gabarit ;
   . un guide de coupe distale, coulissant le long de ladite tige de guidage, et pourvu d'une fente traversante orientée perpendiculairement à ladite tige, et parallèlement à la glissière.

   En d'autres termes, l'invention consiste à proposer un ancillaire modulaire, pour lequel les différentes variables de coupe, c'est à dire de préparation de l'extrémité du fémur, fonctions du patient à opérer, sont déterminées au moyen d'éléments coulissants, les coupes à effectuer étant alors localisées par la mise en place desdits éléments coulissants.
   Selon une forme de réalisation avantageuse de l'invention :
   - l'extrémité supérieure de la glissière comporte des graduations, au niveau desquelles vient se positionner le guide de coupe antérieure,muni d'un repère venant faire face à l'une des graduations, indiquant de la sorte la taille de prothèse à utiliser, et précisant les valeurs, par exemple en millimètres, des coupes osseuses à effectuer sur les condyles postérieurs et distaux du fémur ; de la sorte, on obtient un équilibre de tension des masses ligamentaires en flexion et en extension ;
   - le guide de coupe distale est pourvu d'une découpe en queue d'aronde, destinée à coopérer avec la tige de guidage du guide de coupe antérieure, de forme complémentaire ; ladite tige de guidage comporte en outre des graduations, par exemple millimétriques, permettant de reporter précisément la valeur déterminée précédemment par le guide de coupe antérieure au niveau de la glissière ;
   - le guide de coupe distale est muni d'une pluralité d'orifices, répartis en deux groupes en translation l'un par rapport à l'autre, et destinés à recevoir des vis ou broches pour la fixation dudit guide dans la partie corticale du fémur, et permettant de modifier la valeur de la coupe initialement prévue, en excès ou par défaut, et ceci sans devoir effectuer de nouvelles visées ;
   - le coulisseau comporte deux orifices traversants, symétriques par rapport à l'élément de guidage de la tige centro-médullaire, et destinés à recevoir des vis de compensation de l'éffondrement local des condyles du fémur.

La manière dont l'invention peut être réalisée et les avantages qui en découlent, ressortiront mieux de l'exemple de réalisation qui suit, donné à titre indicatif et non limitatif à l'appui des figures annexées.

La figure 1 est une représentation schématique en perspective de l'ancillaire modulaire conforme à l'invention.

La figure 2 est une représentation schématique d'une vue latérale de l'ancillaire de l'invention.

La figure 3 est une représentation schématique d'une vue latérale de l'ancillaire en place au niveau de l'extrémité inférieure d'un fémur lors de l'une des opérations de préparation.

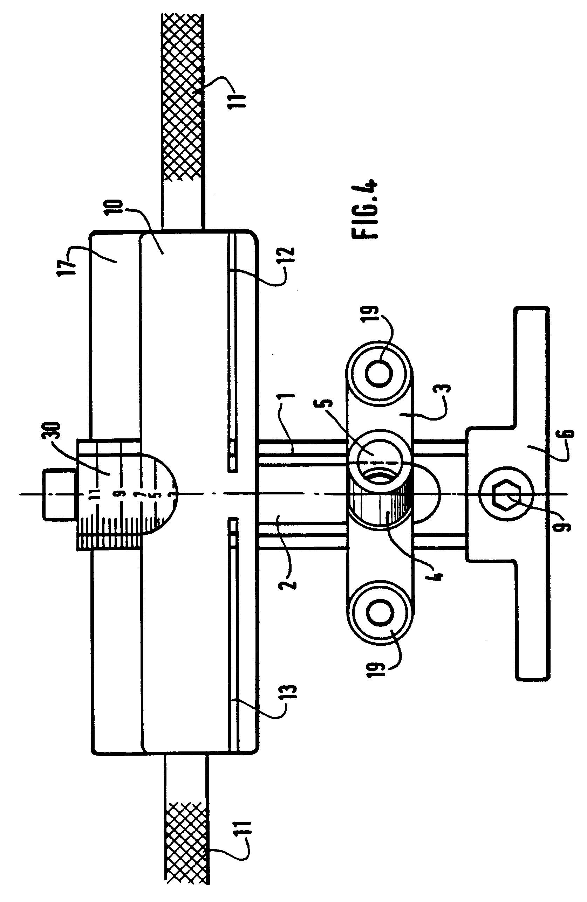

La figure 4 est une vue de face de l'ancillaire.

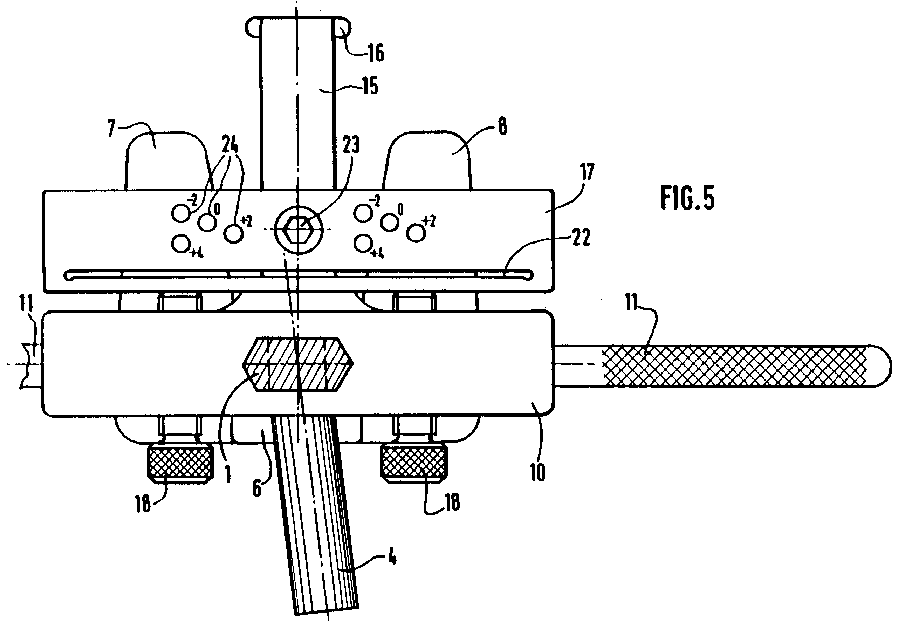

La figure 5 est une vue de dessus de l'ancillaire.

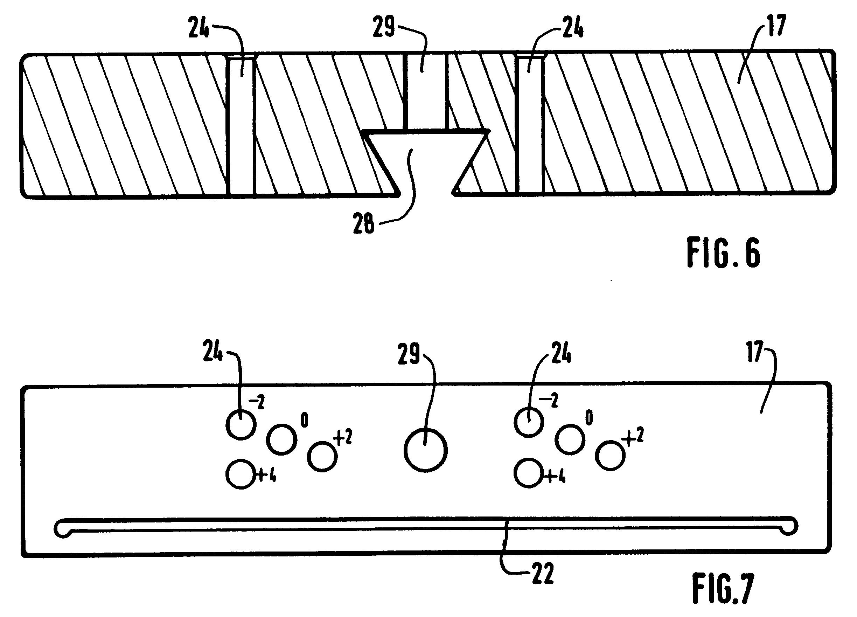

La figure 6 est une vue en coupe transversale du guide de coupe distale.

La figure 7 est une vue de dessus du guide de coupe distale.

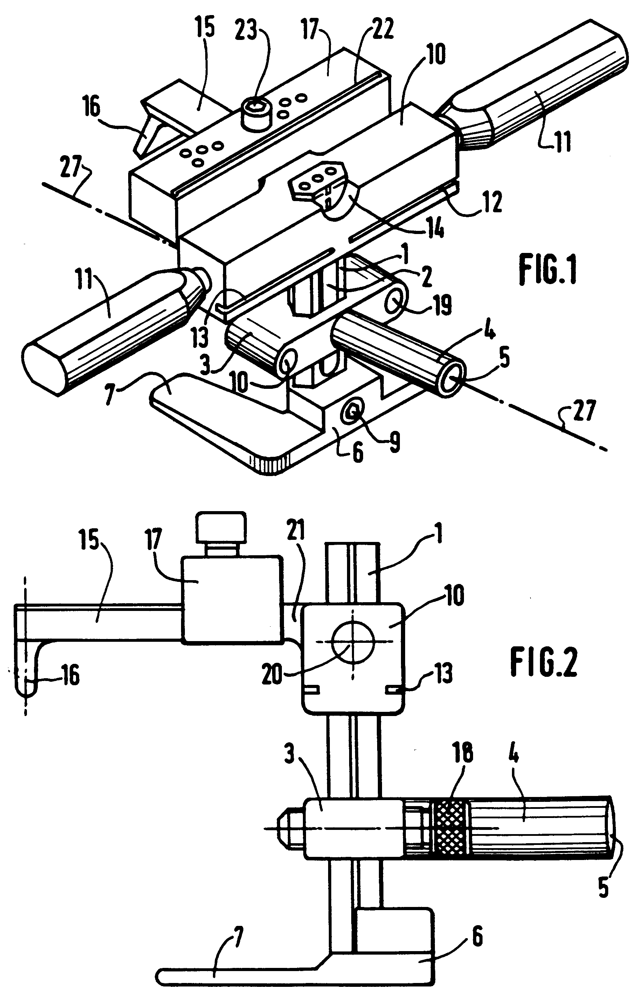

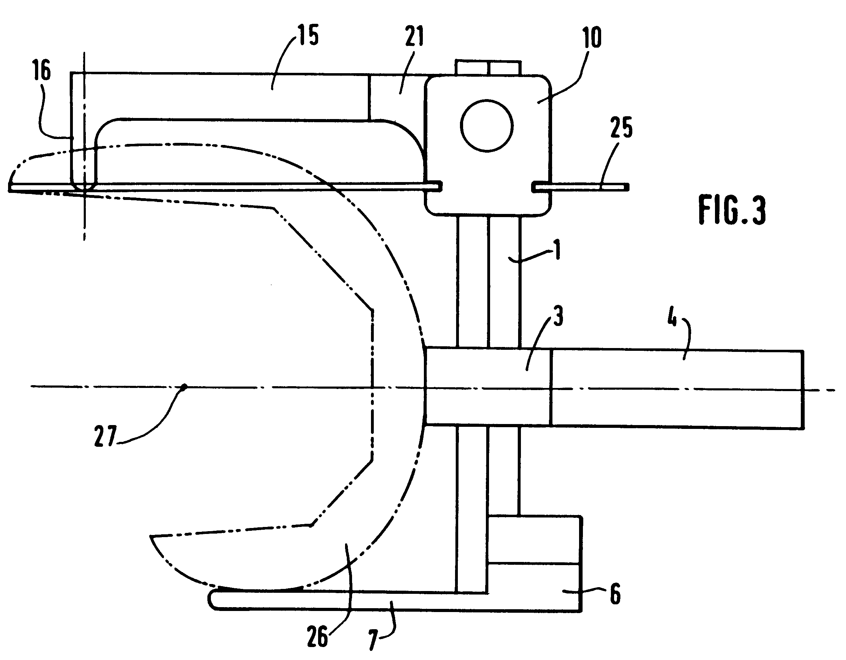

On a donc représenté sur la figure 1 une représentation schématique en perspective de l'ancillaire conforme à l'invention. Fondamentalement, celui-ci est constitué d'une glissière (1), percée en son centre d'une lumière traversante (2), alignée avec la principale dimension de la glissière. La lumière (2) est avantageusement ovalisée à ses deux extrémités. Cette lumière (2) est destinée à coopérer avec un coulisseau (3), dont la fonction est de guider une tige centro-médullaire, représentée schématiquement par le pointillé (27) dans la figure 3. Ce coulisseau (3) comporte en effet un élément creux de guidage (4), traversant ledit coulisseau et débouchant au niveau de la lumière (2) de la glissière (1).

Comme on le sait, le fémur présente par rapport à l'axe mécanique de la jambe, une déviation appelée valgus, s'étalant typiquement selon les individus entre 3 et 9 degrés. De la sorte, il est nécessaire d'en tenir compte lors de la mise en place d'une prothèse du genou. De fait, la tige centro-médullaire (27) doit être guidée pour tenir compte de ce valgus. En conséquence, l'élément de guidage (4) présente une légère inclinaison par rapport à la normale du coulisseau (3). Dans l'exemple représenté, ce coulisseau est adapté pour la mise en place d'un genou droit avec un valgus de 7°.

La glissière (1) comporte à son extrémité inférieure un gabarit dénommé couramment "fourchette de gabarit" (6), comportant deux éléments plans respectivement (7) et (8), perpendiculaires à la glissière (1), et destinés à venir s'appuyer sous les deux condyles respectivement externe et interne du genou lorsque l'articulation est à 90 degrés de flexion. Cette fourchette de gabarit (6) est maintenue solidaire de la glissière (1) au moyen d'une vis (9).

Le coulisseau (3) est pourvu de deux orifices symétriques (19) par rapport au centre de la glissière (1), destinés à recevoir des vis de compensation (18), afin, lors de la mise en place de l'ancillaire par insertion du coulisseau (3) sur la tige centro-médullaire (27), de compenser les défauts des condyles, en prenant appui sur ceux-ci, et ce, pour assurer des découpes correctes et précises du fémur. En outre, on assure de la sorte une stabilisation de l'ancillaire.

L'extrémité supérieure de la glissière (1) reçoit un guide de coupe antérieure primaire (10), susceptible de coulisser sur la glissière (1), et fixable sur cette dernière au moyen de poignées latérales (11). Avantageusement, ce guide de coupe antérieure (10) présente sur sa face antérieure une échancrure, par exemple de forme demi-cylindrique (14), destinée à permettre la lecture de graduations (30) ménagées sur la face antérieure de la partie supérieure de la glissière (1). De la sorte, et comme on le verra ultérieurement, lorsque l'ancillaire est rigoureusement adapté à l'extrémité inférieure du fémur à opérer, le praticien est à même de lire directement sur la graduation la taille de prothèse à utiliser.

Ce guide de coupe antérieure comporte à la partie inférieure de sa face antérieure, deux fentes traversantes respectivement (12) et (13), destinées à permettre le passage d'une scie pour réaliser, comme il sera décrit ultérieurement, l'arrasement de l'os du fémur au niveau de la corticale antérieure.

En outre, le guide de coupe antérieure (10) se prolonge au niveau de sa face postérieure par une tige de guidage (15), comportant à son extrémité libre un palpeur (16), destiné à venir se positionner au contact de la partie corticale antérieure du fémur. L'autre extrémité (21) de la tige (15) est fixée au guide de coupe antérieure (10). Cette tige de guidage (15) est en outre destinée à recevoir un guide de coupe distale (17), susceptible de glisser le long de la tige (15) et d'être fixé également sur cette tige (15), et ce au moyen d'une vis (23), venant s'insérer dans un orifice (29) ménagé dans ledit guide, pour venir au contact de la tige (15). Avantageusement, la face inférieure du guide de coupe distale (17) comporte un évidement (28) en queue d'aronde, destiné à coopérer avec la tige (15), de forme complémentaire. De la sorte, on favorise le coulissement dudit guide et également son maintien.

Le guide de coupe distale (17) comporte également sur sa partie supérieure deux jeux d'orifices traversants (24), chacun des jeux étant en relation de translation par rapport à l'autre, et destinés à permettre la mise en place de broches, pour assurer la fixation du guide de coupe distale (17) au niveau du fémur (26) avant la réalisation de la coupe distale de l'extrémité du fémur à remplacer. Ces orifices (24) vont par paires, et correspondent à des repères permettant d'effectuer une coupe plus ou moins en excès ou au contraire en défaut par rapport à la taille de la prothèse utilisée.

Il va être maintenant décrit plus en détail le mode de fonctionnement de l'ancillaire conforme à l'invention. On procède tout d'abord à l'insertion de la tige centro-médullaire (27) dans le canal médullaire du fémur (26). On insère alors l'ancillaire, par l'intermédiaire du coulisseau (3) sur cette tige, qui émerge par l'ouverture (5) de l'élément de guidage (4), puis on procède à son ajustement sur l'extrémité inférieure du fémur, en faisant reposer les condyles postérieurs du fémur sur la fourchette de gabarit (6,7,8), en faisant jouer les vis de compensation (18), si l'un des deux condyles est particulièrement usé, et en positionnant le palpeur (16) sur la corticale antérieure.

Cet ajustement permet de positionner de manière correcte le guide de coupe antérieure (10), notamment du fait que le palpeur est solidaire de ce dernier par l'intermédiaire de la tige de guidage (15). Corrélativement, il permet alors d'effectuer la lecture en (14) de la graduation (30), indiquant la taille de la prothèse à utiliser. On procède alors au serrage des poignées (11).

Une fois le guide de coupe antérieure (10) mis en place et maintenu par les poignées (11), on procède à la découpe de la corticale antérieure du fémur (26), en introduisant une scie dans chacune des fentes (12) et (13) dudit guide. On obtient ainsi une coupe sensiblement parallèle au canal médullaire, coupe qui vient arraser l'os au niveau de la corticale antérieure.

On procède alors à la mise en place du guide de coupe distale (17) sur la tige de guidage (15), et on le positionne au contact du guide de coupe antérieure (10). Ensuite, le praticien procède à la fixation de ce guide de coupe distale au moyen de broches introduites dans les orifices (24), en excès ou en défaut, voire correspondant à la taille même de la prothèse, selon l'état du genou du patient. Une fois ce guide de coupe distale (17) fixé, on retire l'ensemble de l'ancillaire, tout d'abord en desserrant les poignées latérales (11), puis en faisant coulisser la tige de guidage (15) hors de l'évidement en queue d'aronde (28) dudit guide de coupe distale. On procède alors à la découpe de la partie distale du fémur (26) en introduisant une scie dans la fente (22) dudit guide.

Ensuite, et de manière connue, on utilise des gabarits de coupe adaptés à chaque taille pour effectuer les découpes définitives ainsi que deux chanfreins, destinés à parfaire l'extrémité inférieure du fémur pour recevoir la prothèse de genou.

De la sorte, il ressort de cette invention, qu'un tel ancillaire developpe une grande capacité d'adaptation lors de la mise en place de prothèse de genou, limitant de façon importante le nombre de pièces à utiliser. Il est en outre adaptable à tous types de genou. Comme on l'a dit, cet ancillaire est muni de vis de rattrapage d'usure et de compensation, permettant de s'adapter même à des genoux particulièrement pathologiques. Enfin, l'une des bases de référence pour la mise en place de l'ancillaire étant constituée par la corticale antérieure du fémur à opérer, on observe une plus grande précision dans le travail préparatoire.

## Revendications

1. Ancillaire modulaire pour la mise en place d'une prothèse de genou, se composant:
- d'une glissière rectiligne (1),
- d'un gabarit (6), fixé à l'extrémité inférieure de la glissière (1), et comportant deux éléments plans (7,8) perpendiculaires à la glissière, destinés à venir prendre appui sur les condyles postérieurs du fémur;
- d'un guide de coupe antérieure (10), fixable sur cette ladite glissière, ledit guide comportant:
. au moins une fente (12,13) traversante perpendiculaire à la glissière (1);
. un palpeur cortical (16), ménagé à l'extrémité libre d'une tige de guidage (15) s'étendant parallèlement au gabarit (6,7,8);
**caractérisé en ce que**
- ladite glissière rectiligne (1) est percée en son centre d'une lumière (2) également rectiligne, parallèle à la dimension principale de la glissière, recevant un coulisseau (3) destiné à servir de guide (4) pour une tige centro-médullaire (27) insérée dans le canal médullaire du fémur (26) de l'articulation à opérer, et permettant d'assurer la mise en place de l'ancillaire;
- ledit guide de coupe antérieure (10) est coulissable sur la glissière (1) au dessus du coulisseau (3),
. l'autre extrémité (21) du palpeur cortical (16) est solidaire du guide de coupe antérieure (10),
. ledit guide de coupe antérieure (10) comporte en outre un guide de coupe distale (17), coulissant le long de ladite tige de guidage (15), et pourvu d'une fente traversante (22) orientée perpendiculairement à ladite tige (15), et parallèlement à la glissière (1).

2. Ancillaire selon la revendication 1, caractérisé en ce que l'extrémité supérieure de la glissière (1) comporte des graduations (30), au niveau desquelles vient se positionner le guide de coupe antérieure (10), muni d'un repère (14) venant faire face à l'une des graduations, indiquant de la sorte la taille de prothèse à utiliser, et précisant les valeurs des coupes osseuses à effectuer sur les condyles postérieurs et distaux du fémur.

3. Ancillaire selon la revendication 2, caractérisé en ce que la tige de guidage (15) comporte des graduations exprimées dans une unité identique à celle utilisée pour les graduations (30) de la glissière (1), destinées à permettre le report de la valeur indiquée par le repère (14) pour la mise en place du guide de coupe distale (17).

4. Ancillaire selon l'une des revendications 1 à 3, caractérisé en ce que le guide de coupe distale (17) est pourvu d'une découpe en queue d'aronde (28), destinée à coopérer avec la tige de guidage (15) de forme complémentaire.

5. Ancillaire selon l'une des revendications 1 à 4, caractérisé en ce que le guide de coupe distale (17) est muni d'une pluralité d'orifices (24), répartis selon deux groupes en translation l'un par rapport à l'autre, et destinés à recevoir des vis ou broches pour la fixation dudit guide (17) dans la partie corticale du fémur, et permettant de modifier la valeur de la coupe initialement prévue par excès ou par défaut sans nécessiter une nouvelle visée.

6. Ancillaire selon l'une des revendications 1 à 5, caractérisé en ce que le coulisseau (3) comporte deux orifices traversants (19), symétriques par rapport à l'élément de guidage (4) de la tige centro-médullaire (27), et destinés à recevoir des vis de compensation (18) de l'éffondrement local des condyles du fémur.

## Claims

1. Modular ancillary instrument for fitting a knee prosthesis, composed:
- of a straight slideway (1),
- of a template (6), fixed to the lower end of the slideway (1) and comprising two plane elements (7,8) perpendicular to the slideway which are intended to bear on the posterior condyles of the femur;
- of an anterior sectioning guide (10) which can be fixed onto the said slideway, the said guide comprising:
. at least one through-slot (12,13) perpendicular to the slideway (1);
. a cortical contact element (16), arranged at the free end of a guide rod (15) extending parallel to the template (6, 7, 8);
characterized in that
- the said straight slideway (1) is pierced at its centre by an elongated hole (2), also straight, parallel to the main dimension of the slideway, receiving a slide (3) intended to serve as a guide (4) for a centromedullary rod (27) inserted into the medullary cavity of the femur (26) of the joint which is to be operated on, and making it possible to fit the ancillary instrument;
- the said anterior sectioning guide (10) can be slid over the slideway (1) above the slide (3),
. the other end (21) of the cortical contact element (16) is solidly attached to the anterior sectioning guide (10),
. the said anterior sectioning guide (10) furthermore comprises a distal sectioning guide (17) sliding along the said guide rod (15) and provided with a through-slot (22) oriented perpendicularly to the said rod (15) and parallel to the slideway (1).

2. Ancillary instrument according to Claim 1, characterized in that the upper end of the slideway (1) comprises gradations (30) at which is fitted the anterior sectioning guide (10) provided with a marker (14) which faces one of the gradations, thereby indicating the size of the prosthesis to be used and specifying the values of the bone sections to be made on the posterior and distal condyles of the femur.

3. Ancillary instrument according to Claim 2, characterized in that the guide rod (15) comprises gradations expressed in a unit identical to that used for the gradations (30) on the slideway (1), which are intended to allow reading of the value indicated by the marker (14) for fitting the distal sectioning guide (17).

4. Ancillary instrument according to one of Claims 1 to 3, characterized in that the distal sectioning guide (17) is provided with a dovetailed cut-out (28) intended to cooperate with the guide rod (15) of complementary shape.

5. Ancillary instrument according to one of Claims 1 to 4, characterized in that the distal sectioning guide (17) is provided with a plurality of orifices (24) distributed in two groups in translation with respect to one another and intended to receive screws or pins for fixing the said guide (17) in the cortical part of the femur, and making it possible positively or negatively to alter the value of the section initially foreseen without requiring realignment.

6. Ancillary instrument according to one of Claims 1 to 5, characterized in that the slide (3) comprises two through-orifices (19) which are symmetrical with respect to the guide element (4) of the centromedullary rod (27) and are intended to receive screws (18) for compensating for the local collapse of the condyles of the femur.

## Patentansprüche

1. Modulares Zusatzgerät zum Anbringen einer Knieprothese, welches sich zusammensetzt aus:
- einer geraden Gleitschiene (1),
- einer Schablone (6), die am unteren Ende der Gleitschiene (1) befestigt ist und zwei ebene Elemente (7, 8) aufweist, die rechtwinklig zur Gleitschiene (1) verlaufen und dazu vorgesehen sind, gegen die posterioren Kondylen des Femurs angelegt zu werden;
- einer Führung (10) für einen anterioren Schnitt, welche auf der genannten Gleitschiene befestigbar ist und folgendes umfaßt:
. mindestens einen durchgehenden Schlitz (12, 13), der rechtwinklig zur Gleitschiene (1) verläuft;
. einen kortikalen Taster (16), der am freien Ende einer Führungsstange (15) angeordnet ist, welche sich parallel zu der Schablone (6, 7, 8) erstreckt;
dadurch gekennzeichnet, daß
- die genannte gerade Gleitschiene (1) in ihrer Mitte mit einem ebenfalls geraden Langloch (2) versehen ist, welches parallel zur Hauptabmessung der Gleitschiene verläuft und einen Gleitschieber (3) aufnimmt, der dazu vorgesehen ist als Führung (4) einer zentro-medullären Stange (27), welche in den Knochenmarkskanal des Femurs (26) des zu operierenden Gelenks eingeführt ist, zu dienen und das Anbringen des Zusatzgeräts sicherstellt;
- die genannte anteriore Schnittführung (10) auf der Gleitschiene (1) oberhalb des Gleitschiebers (3) verschiebbar ist, wobei
. das andere Ende (21) des kortikalen Tasters (16) fest mit der anterioren Schnittführung (10) verbunden ist,
. die anteriore Schnittführung (10) weiterhin eine distale Schnittführung (17) aufweist, welche entlang der genannten Führungsstange (15) verschiebbar ist und mit einem durchgehenden Schlitz (22) versehen ist, der rechtwinklig zur genannten Führungsstange (15) und parallel zur Gleitschiene (1) verläuft.

2. Zusatzgerät nach Anspruch 1, dadurch gekennzeichnet, daß das obere Ende der Gleitschiene (1) Maßeinteilungen (30) aufweist, in deren Bereich die anteriore Schnittführung (10) angebracht wird, welche mit einer Markierung (14) versehen ist, die in eine Stellung gegenüber einer der Maßeinteilungen gebracht wird und so die Größe der zu verwendenden Prothese anzeigt und die Werte der auf den posterioren und distalen Kondylen des Femurs durchzuführenden Knochenschnitte präzisiert.

3. Zusatzgerät nach Anspruch 2, dadurch gekennzeichnet, daß die Führungsstange (15) Maßeinteilungen in einer Einheit aufweist, die mit der Einheit der Maßeinteilungen (30) der Gleitschiene (1) identisch ist, welche dazu vorgesehen ist, die Übertragung des durch die Markierung (14) angezeigten Werts zum Anbringen der distalen Schnittführung (17) zu ermöglichen.

4. Zusatzgerät nach einem der Ansprüche 1 bis 3, dadurch gekennzeichnet, daß die distale Schnittführung (17) mit einer schwalbenschwanzförmigen Ausnehmung (28) versehen ist, die dazu vorgesehen ist, mit der komplementär geformten Führungsstange (15) zusammen zu wirken.

5. Zusatzgerät nach einem der Ansprüche 1 bis 4, dadurch gekennzeichnet, daß die distale Schnittführung (17) mit einer Vielzahl von Bohrungen versehen ist, welche in zwei zueinander parallel verschobenen Gruppen angeordnet sind und dazu vorgesehen sind, Schrauben oder Stifte für die Befestigung der genannten distalen Schnittführung (17) in dem kortikalen Bereich des Femurs aufzunehmen und eine Veränderung des Wertes des ursprünglich vorgesehenen Schnittes nach oben oder unten ohne Notwendigkeit eines neuen Ausrichtens zu ermöglichen.

6. Zusatzgerät nach einem der Ansprüche 1 bis 5, dadurch gekennzeichnet, daß der Gleitschieber (3) zwei durchgehende Öffnungen (19) aufweist, die bezüglich des Führungselements (4) für die zentro-medulläre Stange (27) symmetrisch sind und dazu vorgesehen sind, Ausgleichsschrauben (18) zum Ausgleich der lokalen Einsenkungen der Kondylen des Femurs aufzunehmen.
